# EUROPEAN PATENT SPECIFICATION

(11) **EP 4 171 441 B1**
(45) Date of publication and mention of the grant of the patent: **12.11.2025**
(21) Application number: 20742563.8
(22) Date of filing: 25.06.2020
(51) Int. Cl.: A61F 2/04, A61F 2/06

(54) **LYMPH CONDUCTION SYSTEM IMPLANT**
IMPLANT FÜR DAS LYMPH-KONTROLLSYSTEM
IMPLANT POUR LE SYSTÈME DE CONDUCTION LYMPHATIQUE

(43) Date of publication of application: 03.05.2023
(73) Proprietor: Bard Peripheral Vascular, Inc., Franklin Lakes, NJ 07417 (US)
(72) Inventor: FORD, Summer, Franklin Lakes, NJ 07417 (US)
(74) Representative: Hoffmann Eitle
(86) International application number: PCT/US2020/039595
(87) International publication number: WO 2021/262176

(56) References cited:
- WO-A1-2006/116636
- WO-A1-2015/130184
- WO-A1-2017/015571
- US-A1- 2004 167 613
- US-A1- 2020 054 867

## Description

### Technical Field

The present invention relates to a device for use in a lymphatic system of a patient, and, more particularly, to a lymph conduction system implant.

### Background Art

As cancer metastasizes from the original tumor into surrounding tissue, cancerous cells often become trapped within the lymph nodes of the patient. As a result, a physician can dissect one or more lymph nodes to thereby provide a diagnostic benefit of understanding whether or not the cancer has metastasized from the surrounding tissue, and to what extent. However, there may be adverse effects associated with lymph node dissection including, but not limited to, leaving the patient with a predisposition for infection, lymphoedema, oedema or seroma.

US 2020/054867 relates to a method for removing excess fluid from a lymphatic system in which a passage is created between the lymphatic system and an external drainage device. The device is in the form of a stent-graft having a non-porous side wall having a proximal and a distal end, wherein all exposed surfaces are made of a first biocompatible material, and comprises a porous membrane connected to the proximal end of the stent-graft and configured to span the inflow port of the lumen of the stent-graft, the porous membrane made of a second biocompatible material.
WO 2017/015571 relates to an implantable drug delivery device comprising hollow fiber walls for releasing a drug. WO 2006/11663 relates to a vascular intervention device comprising a stent with a filter at the distal end of the stent. WO 2015/130184 relates to a stent for catching and breaking up of thromboemboli. The stent comprises nitinol meshes in a plastic tube and microchips coupled to the plastic tube.

What is needed in the art is a device that will help negate the adverse effects following lymph node dissection by helping to restore proper conduction of lymph fluid at the site of lymph node dissection.

### Summary of Invention

The present invention provides a device that will help negate the adverse effects following lymph node dissection by helping to restore proper conduction of lymph fluid at the site of lymph node dissection.

The invention, in one form, is directed to a lymph conduction system implant for implantation in a lymphangion, and that includes a stent graft and a porous membrane. The stent graft has a non-porous tubular side wall. The non-porous tubular side wall has a proximal end and a distal end. The non-porous tubular side wall is configured to define a lumen having an inflow port at the proximal end and an outflow port at the distal end. All exposed surfaces of the stent graft are made of a first biocompatible material. The porous membrane is connected to the proximal end of the non-porous tubular side wall of the stent graft. The porous membrane is configured to span an entirety of a transverse area of the inflow port of the lumen of the stent graft. The porous membrane is made of a second biocompatible material. A length of the implant is no more than one-half of an overall length of the lymphangion.

The invention, in another form, is directed to a lymph conduction system implant for implantation in a lymphangion, and that includes a stent graft, a drug eluting porous membrane, and a drug. The stent graft has a non-porous tubular side wall. The non-porous tubular side wall has an inferior lymphangion region end and a superior lymphangion region end. The non-porous tubular side wall is configured to define a lumen having an inflow port at the inferior lymphangion region end and an outflow port at the superior lymphangion region end. All exposed surfaces of the stent graft are made of a first biocompatible material. A drug eluting porous membrane is connected to the inferior lymphangion region end of the non-porous tubular side wall of the stent graft. The drug eluting porous membrane is configured to span an entirety of a transverse area of the inflow port of the lumen of the stent graft. The drug eluting porous membrane is made of a second biocompatible material. The drug is carried by the drug eluting porous membrane. A length of the implant is no more than one-half of an overall length of the lymphangion.

An advantage of the present invention is that the lymph conduction system implant helps to negate the adverse effects following lymph node dissection by helping to restore proper conduction of lymph fluid at the site of lymph node dissection.

Another advantage is that the drug eluting porous membrane may provide localized and/or systemic drug delivery with sustained/prolonged release.

### Brief Description of Drawings

The above-mentioned and other features and advantages of this invention, and the manner of attaining them, will become more apparent and the invention will be better understood by reference to the following description of an embodiment of the invention taken in conjunction with the accompanying drawings, wherein:
Fig. 1 is a perspective view of a lymph conduction system implant in accordance with an aspect of the present invention;
Fig. 2 is a section view of the lymph conduction system implant of Fig. 1 taken along plane 2-2-2-2 of Fig. 1; and
Fig. 3 is a pictorial illustration of the lymph conduction system implant of Figs. 1 and 2 that is positioned in a lymphatic vessel of the lymphatic system in accordance with an aspect of the present invention.

Corresponding reference characters indicate corresponding parts throughout the several views. The exemplifications set out herein illustrate at least one embodiment of the invention, and such exemplifications are not to be construed as limiting the scope of the invention in any manner.

### Description of Embodiments

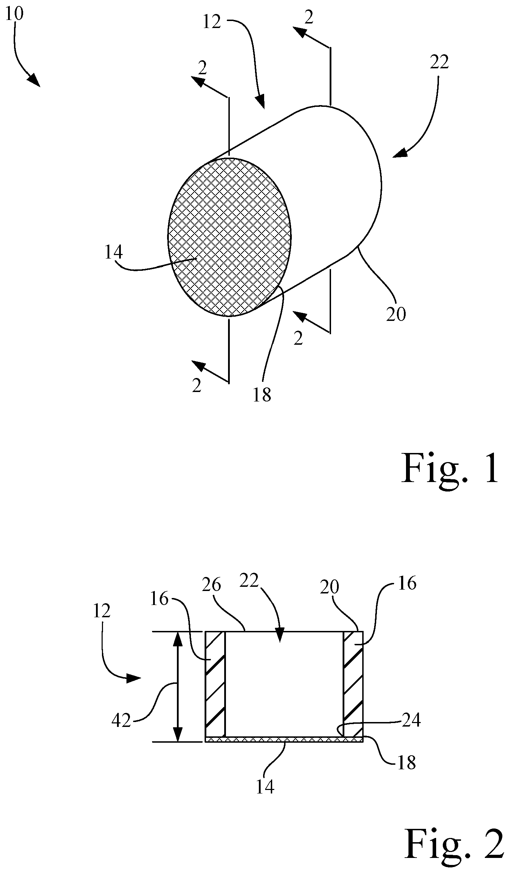

Referring now to the drawings, and more particularly to Figs. 1 and 2, there is shown a lymph conduction system implant 10 in accordance with an aspect of the present invention.

Lymph conduction system implant 10 includes a stent graft 12 and a porous membrane 14. Stent graft 12 has a non-porous tubular side wall 16. Non-porous tubular side wall 16 has a proximal end 18 and a distal end 20. Functionally, proximal end 18 also may be described herein as inferior lymphangion region end 18 of lymph conduction system implant 10, and distal end 20 also may be described herein as superior lymphangion region end 20 of lymph conduction system implant 10.

Non-porous tubular side wall 16 may be in the form of a polymer tubular member that defines a lumen 22 having an inflow port 24 at proximal end 18 and an outflow port 26 at distal end 20. The transverse cross-sectional shape of the polymer tubular member, and in turn lumen 22, may be, for example, circular or elliptical. Stent graft 12 may have a diameter that is an approximation of the diameter of the lymphatic vessel size into which lymph conduction system implant 10 is to be inserted, e.g., a diameter in a range of 0.2 millimeters (mm) to 2 mm, so as to provide a snug fit in the lymphatic vessel.

All exposed surfaces of stent graft 12 are made of a first biocompatible material, such as for example, expanded polytetrafluoroethylene (ePTFE). In the present embodiment, non-porous tubular side wall 16 includes ePTFE, such as for example, wherein non-porous tubular side wall 16 of stent graft 12 includes a coating of ePTFE. For example, the coating of ePTFE may be used to achieve the non-porosity of non-porous tubular side wall 16.

Porous membrane 14 may be connected, e.g., by ultrasonic or other weld, hot melt adhesive, or overmolding, to proximal end 18 of non-porous tubular side wall 16 of stent graft 12. Also, optionally, porous membrane 14 may be formed by direct electrospinning onto a surface of a polymer sheet, wherein the polymer sheet may be attached to stent graft 12 (e.g., via hot melt adhesive, direct wire attachment, or overmold) either before or after the electrospinning of porous membrane 14 onto the polymer sheet surface.

Porous membrane 14 is configured, in size and shape, to span an entirety of a transverse area of inflow port 24 of lumen 22 of stent graft 12. Porous membrane 14 may have a thickness, for example, in a range of 50 nanometers (nm) to 100 nm. Porous membrane 14 may be made of a second biocompatible material, and also may be made of a resorbable material. For example, the second biocompatible material of porous membrane 14 may include, in whole or in part, polyglycolide or polyglycolic acid (PGA) or polylactic-co-glycolic acid (PLGA). In the present embodiment, porous membrane 14 is a mesh of electrospun fibers that is coupled to proximal end 18 of non-porous tubular side wall 16 of stent graft 12, e.g., by one of the connection methods described above .

Optionally, porous membrane 14 may be configured as a drug eluting porous membrane that carries a drug. The drug may be, for example, an antibacterial drug, a chemo-therapeutic drug, and/or an anti-metastasis drug. The drug may be impregnated on and between the electrospun fibers making up porous membrane 14. Accordingly, for purposes of the drug eluting option, porous membrane 14 may sometimes also be referred to as drug eluting porous membrane 14.

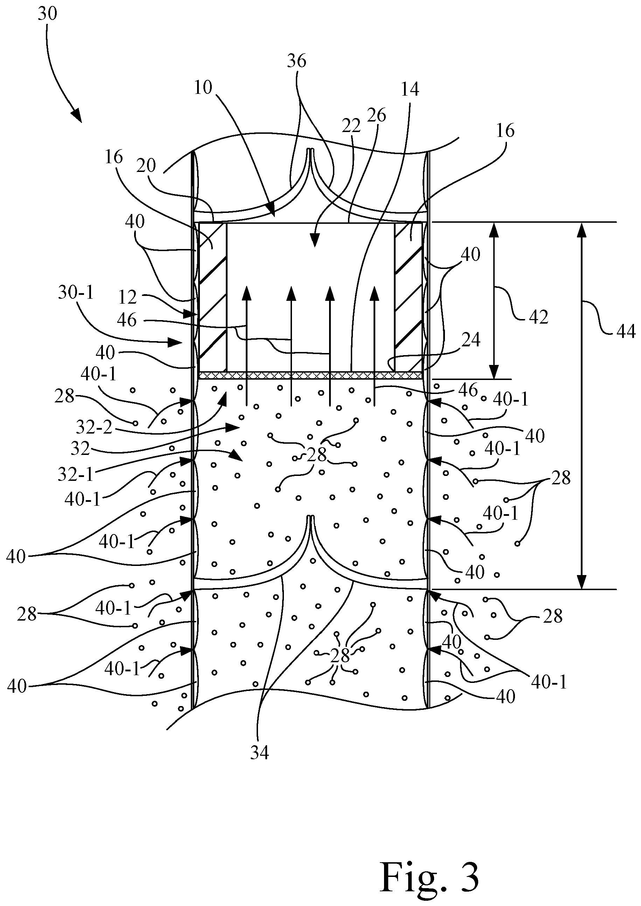

Referring also to Fig. 3, lymph conduction system implant 10 is configured to facilitate conduction of lymph fluid 28 (represented in Fig. 3 as small circles) in a lymphatic vessel 30 following lymph node removal, or lymph node dissection, as a result of cancer metastasis or other causes. Lymph conduction system implant 10 may be deployed into the lymphatic system with a standard stent graft balloon system, placing lymph conduction system implant 10 at a site 30-1 of lymph node dissection. The method of use includes positioning lymph conduction system implant 10 in lymphatic vessel 30 in a lymphangion 32 between an inferior (entry) valve 34 in (i.e., at the proximal end of) an inferior lymphangion region 32-1 of lymphangion 32 and a superior (exit) valve 36 in (i.e., at a distal end of) a superior lymphangion region 32-2 of lymphangion 32. In the orientation of lymphangion 32 depicted in Fig. 3, inferior lymphangion region 32-1 may be considered to be the lower portion (e.g., lower half) of lymphangion 32 and superior lymphangion region 32-2 may be considered to be the upper portion (e.g., upper half) of lymphangion 32. Each of inferior (entry) valve 34 and superior (exit) valve 36 is a one-way bicuspid valve that prevents backwards drainage of lymph fluid 28.

The primary functionality of lymph conduction system implant 10 is that of restoring conduction of lymph fluid 28 to aid in preventing lymphoedema, seroma, or oedema following lymph node dissection. More particularly, distal end 20 (e.g., the open end) of stent graft 12 is positioned adjacent the superior (exit) valve 36 in superior lymphangion region 32-2 and proximal end 18 of stent graft 12 (e.g., the covered end) is positioned with porous membrane 14 facing the inferior (entry) valve 34 in inferior lymphangion region 32-1, so as to generate a pressure difference as well as a concentration gradient as an influx of lymph fluid 28 enters inferior lymphangion region 32-1 at a location proximal to lymph conduction system implant 10 through junctions 40-1 (represented by curved arrows) between endothelial cells 40 that are loosely layered and make up lymphatic vessel(s) 30, allowing flow of lymph fluid 28 into each lymphangion 32.

To facilitate conduction of lymph fluid 28, a length 42 of lymph conduction system implant 10 is no more than one-half of an overall length 44 of lymphangion 32. Stated differently, the length 42 of lymph conduction system implant 10 is no more than one-half of a distance (corresponding to overall length 44) between the inferior (entry) valve 34 in inferior lymphangion region 32-1 and the superior (exit) valve 36 in superior lymphangion region 32-2. For example, since an average length of lymphangion 32 may be approximately 2 mm, then the average length of stent graft 12 may be approximately less than or equal to 1 mm.

Accordingly, length 42 of lymph conduction system implant 10 is selected to allow for the superior region walls of superior lymphangion region 32-2 to be blocked off by non-porous tubular side wall 16 of stent graft 12 of lymph conduction system implant 10, while the inferior half (inferior lymphangion region 32-1) will remain uncovered, thereby allowing for natural influx of lymph fluid 28 into inferior lymphangion region 32-1 of lymphangion 32. This natural influx of lymph fluid 28 is enabled by the endothelium, a general term for the inner layer of a vessel, that is composed of an inner lining of single, flattened epithelial cells (simple squamous epithelium), i.e., endothelial cells 40. The endothelium has junctions 40-1 between endothelial cells 40 that allow interstitial lymph fluid 28 to flow into lymphatic vessel 30 when the pressure becomes high enough (such as from blood capillary hydrostatic pressure), but does not normally allow lymph fluid 28 to leak back out into the interstitial space. By blocking off superior lymphangion region 32-2 of lymphangion 32, and allowing inferior lymphangion region 32-1 of lymphangion 32 to remain unobstructed, a pressure difference, as well as a concentration gradient, will be created as an influx of lymph fluid 28 enters inferior lymphangion region 32-1 of lymphangion 32 through the junctions 40-1 between endothelial cells 40.

The natural conduction of lymph fluid 28 through lymphatic vessel(s) 30 result from the opening and closing of the one-way inferior (entry) valve 34 in inferior lymphangion region 32-1 and the superior (exit) valve 36 in superior lymphangion region 32-2 of lymphangion 32, wherein the opening and closing of the valves is induced primarily by smooth muscle contractions as well as secondary skeletal muscle contraction. This will additionally prevent lymph fluid 28 from flowing backwards. Once inferior (entry) valve 34 opens, lymph fluid 28 will flow into inferior lymphangion region 32-1, in addition to the lymph fluid 28 entering through the endothelial walls of lymphatic vessel 30. Once inferior lymphangion region 32-1 is saturated with lymph fluid 28, inferior (entry) valve 34 will be forced to close due to the fluid pressure distal to inferior (entry) valve 34.

Due to the inability of lymph fluid 28 to enter superior lymphangion region 32-2 through the endothelial walls of lymphatic vessel 30 due to obstruction by non-porous tubular side wall 16 of stent graft 12 of lymph conduction system implant 10, this blockage will create a negative pressure and/or a concentration gradient between inferior lymphangion region 32-1 and superior lymphangion region 32-2 of lymphangion 32. Due to this difference, lymph fluid 28 in inferior lymphangion region 32-1 will flow through porous membrane 14 through passive diffusion, represented by straight arrows 46, flowing into superior lymphangion region 32-2, creating an equilibrium within lymphangion 32. Once lymphangion 32 has met an equilibrium, pressure will begin to build as lymph fluid 28 is able to enter into inferior lymphangion region 32-1 as the junctions 40-1 between endothelial cells 40 are not blocked, allowing a pressure and/or concentration gradient to build up and continuously diffuse across porous membrane 14 until superior (exit) valve 36 gives way to the pressure and/or is opened via smooth muscle contraction. Once superior (exit) valve 36 opens, this allows for adequate conduction of lymph fluid 28 into the remaining lymphatic system and subsequent circulatory system.

The following items also relate to the invention.

In one embodiment, the invention relates to a lymph conduction system implant for implantation in a lymphangion, and that includes a stent graft and a porous membrane. The stent graft has a non-porous tubular side wall. The non-porous tubular side wall has a proximal end and a distal end. The non-porous tubular side wall may be configured to define a lumen having an inflow port at the proximal end and an outflow port at the distal end. All exposed surfaces of the stent graft are made of a first biocompatible material. The porous membrane may be connected to the proximal end of the non-porous tubular side wall of the stent graft. The porous membrane may be configured to span an entirety of a transverse area of the inflow port of the lumen of the stent graft. The porous membrane may be made of a second biocompatible material, which may be different from the first biocompatible material. A length of the implant is no more than one-half of an overall length of the lymphangion.

In some embodiments, the second biocompatible material of the porous membrane may be a resorbable material.

In some embodiments, the second biocompatible material of the porous membrane may include PGA or PLGA.

In some embodiments, the porous membrane may be a mesh of electrospun fibers.

In some embodiments, the non-porous tubular side wall may include ePTFE.

In some embodiments, the non-porous tubular side wall may include a coating of ePTFE.

Optionally, the proximal end may be an inferior lymphangion region end. Optionally, the distal end may be a superior lymphangion region end. Optionally, a length of the lymph conduction system implant is no more than one-half of a distance between an entry valve in an inferior lymphangion region and the exit valve in the superior lymphangion region. Optionally, in some embodiments, the porous membrane may be a drug eluting porous membrane that carries a drug.

In embodiments having a drug, the drug may be an antibacterial drug, a chemo-therapeutic drug, and/or an anti-metastasis drug.

A method of using the lymph conduction system implant according to any one of the above-mentioned embodiments of paragraphs 0029-0036 may facilitate lymph fluid conduction in a lymphatic vessel following lymph node removal, and includes the step of positioning the lymph conduction system implant in the lymphatic vessel in a lymphangion between an entry valve in an inferior lymphangion region of the lymphangion and an exit valve in a superior lymphangion region of the lymphangion and, wherein the distal end of the stent graft may be positioned adjacent the exit valve in the superior lymphangion region and the proximal end of the stent graft may be positioned with the porous membrane facing the entry valve in the inferior lymphangion region, so as to generate a pressure difference as well as a concentration gradient as an influx of lymph fluid enters the inferior lymphangion region at a location proximal to the lymph conduction system implant through the junctions between endothelial cells of the lymphatic vessel.

In the method of using the lymph conduction system implant, a length of the lymph conduction system implant may be no more than one-half of an overall length of the lymphangion.

In the method of using the lymph conduction system implant, a length of the lymph conduction system implant may be no more than one-half of a distance between the entry valve in the inferior lymphangion region and the exit valve in the superior lymphangion region.

In the method of using the lymph conduction system implant, the method may optionally include the further step of re-charging the porous membrane with an antibacterial drug, a chemo-therapeutic drug, and/or an anti-metastasis drug.

In another embodiment, the invention relates to a lymph conduction system implant for implantation in a lymphangion, and having a stent graft, a drug eluting porous membrane, and a drug. The stent graft has a non-porous tubular side wall. The non-porous tubular side wall may have an inferior lymphangion region end and a superior lymphangion region end. The non-porous tubular side wall may be configured to define a lumen having an inflow port at the inferior lymphangion region end and an outflow port at the superior lymphangion region end. All exposed surfaces of the stent graft may be made of a first biocompatible material. The drug eluting porous membrane may be connected to the inferior lymphangion region end of the non-porous tubular side wall of the stent graft. The drug eluting porous membrane may be configured to span an entirety of a transverse area of the inflow port of the lumen of the stent graft. The drug eluting porous membrane may be made of a second biocompatible material, which may be different from the first biocompatible material. The drug may be carried by the drug eluting porous membrane. A length of the implant is no more than one-half of an overall length of the lymphangion.

In some embodiments, the second biocompatible material of the porous membrane may be a resorbable material that includes PGA or PLGA.

In some embodiments, the porous membrane may be a mesh of electrospun fibers.

In any embodiment having a drug, the drug may be an antibacterial drug, a chemo-therapeutic drug, and/or an anti-metastasis drug.

In some embodiments, the non-porous tubular side wall may include ePTFE.

In some embodiments, the non-porous tubular side wall may include a coating of ePTFE.

A method of using the lymph conduction system implant according to any one of the above-mentioned embodiments of paragraphs 0041-0046 may facilitate lymph fluid conduction in a lymphatic vessel following lymph node removal, and includes the step of positioning the lymph conduction system implant in the lymphatic vessel in a lymphangion between an entry valve in an inferior lymphangion region of the lymphangion and an exit valve in a superior lymphangion region of the lymphangion and, wherein the superior lymphangion region end of the stent graft may be positioned adjacent the exit valve in the superior lymphangion region and the inferior lymphangion region end of the stent graft may be positioned with the drug eluting porous membrane facing the entry valve in the inferior lymphangion region, so as to generate a pressure difference as well as a concentration gradient as an influx of lymph fluid enters the inferior lymphangion region at a location proximal to the lymph conduction system implant through the junctions between endothelial cells of the lymphatic vessel.

In the method of paragraph 0047, a length of the lymph conduction system implant may be no more than one-half of the overall length of the lymphangion.

While this invention has been described with respect to at least one embodiment, the present invention can be further modified within the scope of this disclosure. This application is therefore intended to cover any variations, or adaptations of the invention using its general principles. as come within known or customary practice in the art to which this invention pertains and which fall within the limits of the appended claims.

## Claims

1. A lymph conduction system implant (10) for implantation in a lymphangion, comprising:
a stent graft (12) having a non-porous tubular side wall (16), the non-porous tubular side wall having a proximal end (18) and a distal end (20), the non-porous tubular side wall configured to define a lumen (22) having an inflow port (24) at the proximal end and an outflow port (26) at the distal end, and wherein all exposed surfaces of the stent graft are made of a first biocompatible material; and
a porous membrane (14) connected to the proximal end of the non-porous tubular side wall of the stent graft, the porous membrane configured to span an entirety of a transverse area of the inflow port of the lumen of the stent graft, the porous membrane being made of a second biocompatible material;
wherein a length (42) of the implant is no more than one-half of an overall length (44) of the lymphangion.

2. The lymph conduction system implant according to claim 1, wherein the second biocompatible material of the porous membrane is a resorbable material.

3. The lymph conduction system implant according to any one of claims 1 to 2, wherein the second biocompatible material of the porous membrane includes PGA or PLGA.

4. The lymph conduction system implant according to any one of claims 1 to 3, wherein the porous membrane (14) is a mesh of electrospun fibers.

5. The lymph conduction system implant according to any one of claims 1 to 4, wherein the non-porous tubular side wall (16) includes ePTFE.

6. The lymph conduction system implant according to any one of claims 1 to 5, wherein the non-porous tubular side wall (16) includes a coating of ePTFE.

7. The lymph conduction system implant according to any one of claims 1 to 6, wherein the porous membrane (14) sis a drug eluting porous membrane that carries a drug.

8. The lymph conduction system implant according to claim 7, wherein the drug is an antibacterial drug, a chemo-therapeutic drug, and/or an anti-metastasis drug.

9. The lymph conduction system implant (10) according to claim 1, wherein:
the porous membrane is a drug eluting porous membrane (14) connected to the inferior lymphangion region end of the non-porous tubular side wall of the stent graft, the drug eluting porous membrane configured to span an entirety of a transverse area of the inflow port of the lumen of the stent graft, the drug eluting porous membrane being made of a second biocompatible material; and
wherein a drug that is carried by the drug eluting porous membrane.

10. The lymph conduction system implant according to claim 9, wherein the second biocompatible material of the porous membrane is a resorbable material that includes PGA or PLGA.

11. The lymph conduction system implant according to any one of claims 9 to 10, wherein the porous membrane (14) is a mesh of electrospun fibers.

12. The lymph conduction system implant according to any one of claims 9 to 11, wherein the drug is an antibacterial drug, a chemo-therapeutic drug, and/or an anti-metastasis drug.

13. The lymph conduction system implant according to any one of claims 9 to 12, wherein the non-porous tubular side wall (16) includes ePTFE.

14. The lymph conduction system implant according to any one of claims 9 to 13, wherein the non-porous tubular side wall (16) includes a coating of ePTFE.

## Patentansprüche

1. Implantat für das Lymph-Kontrollsystem (10) zur Implantation in ein Lymphangion, umfassend:
einen Stentgraft (12) mit einer nicht-porösen röhrenförmigen Seitenwand (16), wobei die nicht-poröse röhrenförmige Seitenwand ein proximales Ende (18) und ein distales Ende (20) aufweist, wobei die nicht-poröse röhrenförmige Seitenwand so konfiguriert ist, dass sie ein Lumen (22) mit einer Einströmöffnung (24) an dem proximalen Ende und einer Ausströmöffnung (26) an dem distalen Ende definiert, und wobei alle freiliegenden Oberflächen des Stentgrafts aus einem ersten biokompatiblen Material hergestellt sind; und
eine poröse Membran (14), die mit dem proximalen Ende der nicht-porösen röhrenförmigen Seitenwand des Stentgrafts verbunden ist, wobei die poröse Membran so konfiguriert ist, dass sie eine gesamte Querfläche der Einströmöffnung des Lumens des Stentgrafts überspannt, wobei die poröse Membran aus einem zweiten biokompatiblen Material hergestellt ist;
wobei eine Länge (42) des Implantats nicht mehr als die Hälfte einer Gesamtlänge (44) des Lymphangions beträgt.

2. Implantat für das Lymph-Kontrollsystem nach Anspruch 1, wobei das zweite biokompatible Material der porösen Membran ein resorbierbares Material ist.

3. Implantat für das Lymph-Kontrollsystem nach einem der Ansprüche 1 bis 2, wobei das zweite biokompatible Material der porösen Membran PGA oder PLGA einschließt.

4. Implantat für das Lymph-Kontrollsystem nach einem der Ansprüche 1 bis 3, wobei die poröse Membran (14) ein Netz aus elektrogesponnenen Fasern ist.

5. Implantat für das Lymph-Kontrollsystem nach einem der Ansprüche 1 bis 4, wobei die nicht-poröse röhrenförmige Seitenwand (16) ePTFE einschließt.

6. Implantat für das Lymph-Kontrollsystem nach einem der Ansprüche 1 bis 5, wobei die nicht-poröse röhrenförmige Seitenwand (16) eine Beschichtung aus ePTFE einschließt.

7. Implantat für das Lymph-Kontrollsystem nach einem der Ansprüche 1 bis 6, wobei die poröse Membran (14) eine medikamenteneluierende poröse Membran ist, die ein Medikament trägt.

8. Implantat für das Lymph-Kontrollsystem nach Anspruch 7, wobei das Medikament ein antibakterielles Medikament, ein chemotherapeutisches Medikament und/oder ein Anti-Metastasen-Medikament ist.

9. Implantat für das Lymph-Kontrollsystem (10) nach Anspruch 1, wobei:
die poröse Membran eine medikamenteneluierende poröse Membran (14) ist, die mit dem unteren Ende der Lymphangion-Region der nicht-porösen röhrenförmigen Seitenwand des Stentgrafts verbunden ist, wobei die medikamenteneluierende poröse Membran so konfiguriert ist, dass sie eine gesamte Querfläche der Einströmöffnung des Lumens des Stentgrafts überspannt, wobei die medikamenteneluierende poröse Membran aus einem zweiten biokompatiblen Material hergestellt ist; und
wobei ein Medikament, das von der medikamenteneluierenden porösen Membran getragen wird.

10. Implantat für das Lymph-Kontrollsystem nach Anspruch 9, wobei das zweite biokompatible Material der porösen Membran ein resorbierbares Material ist, das PGA oder PLGA einschließt.

11. Implantat für das Lymph-Kontrollsystem nach einem der Ansprüche 9 bis 10, wobei die poröse Membran (14) ein Netz aus elektrogesponnenen Fasern ist.

12. Implantat für das Lymph-Kontrollsystem nach einem der Ansprüche 9 bis 11, wobei das Medikament ein antibakterielles Medikament, ein chemotherapeutisches Medikament und/oder ein Anti-Metastasen-Medikament ist.

13. Implantat für das Lymph-Kontrollsystem nach einem der Ansprüche 9 bis 12, wobei die nicht-poröse röhrenförmige Seitenwand (16) ePTFE einschließt.

14. Implantat für das Lymph-Kontrollsystem nach einem der Ansprüche 9 bis 13, wobei die nicht-poröse röhrenförmige Seitenwand (16) eine Beschichtung aus ePTFE einschließt.

## Revendications

1. Implant de système de conduction lymphatique (10) pour implantation dans un lymphangion, comprenant :
une endoprothèse (12) présentant une paroi latérale tubulaire non poreuse (16), la paroi latérale tubulaire non poreuse présentant une extrémité proximale (18) et une extrémité distale (20), la paroi latérale tubulaire non poreuse étant configurée pour définir une lumière (22) présentant un orifice d'entrée (24) au niveau de l'extrémité proximale et un orifice de sortie (26) au niveau de l'extrémité distale, et dans lequel toutes les surfaces exposées de l'endoprothèse sont fabriquées en un premier matériau biocompatible ; et
une membrane poreuse (14) reliée à l'extrémité proximale de la paroi latérale tubulaire non poreuse de l'endoprothèse, la membrane poreuse étant configurée pour recouvrir une entièreté d'une zone transversale de l'orifice d'entrée de la lumière de l'endoprothèse, la membrane poreuse étant fabriquée en un second matériau biocompatible ;
dans lequel une longueur (42) de l'implant n'est pas plus que la moitié d'une longueur totale (44) du lymphangion.

2. Implant de système de conduction lymphatique selon la revendication 1, dans lequel le second matériau biocompatible de la membrane poreuse est un matériau résorbable.

3. Implant de système de conduction lymphatique selon l'une quelconque des revendications 1 à 2, dans lequel le second matériau biocompatible de la membrane poreuse inclut du PGA ou du PLGA.

4. Implant de système de conduction lymphatique selon l'une quelconque des revendications 1 à 3, dans lequel la membrane poreuse (14) est une maille de fibres électrofilées.

5. Implant de système de conduction lymphatique selon l'une quelconque des revendications 1 à 4, dans lequel la paroi latérale tubulaire non poreuse (16) inclut de l'ePTFE.

6. Implant de système de conduction lymphatique selon l'une quelconque des revendications 1 à 5, dans lequel la paroi latérale tubulaire non poreuse (16) inclut un revêtement d'ePTFE.

7. Implant de système de conduction lymphatique selon l'une quelconque des revendications 1 à 6, dans lequel la membrane poreuse (14) est une membrane poreuse à élution médicamenteuse qui transporte un médicament.

8. Implant de système de conduction lymphatique selon la revendication 7, dans lequel le médicament est un médicament antibactérien, un médicament chimiothérapeutique et/ou un médicament antimétastasique.

9. Implant de système de conduction lymphatique (10) selon la revendication 1, dans lequel :
la membrane poreuse est une membrane poreuse (14) à élution médicamenteuse reliée à l'extrémité de la région du lymphangion inférieur de la paroi latérale tubulaire non poreuse de l'endoprothèse, la membrane poreuse à élution médicamenteuse étant configurée pour recouvrir une entièreté d'une zone transversale de l'orifice d'entrée de la lumière de l'endoprothèse, la membrane poreuse à élution médicamenteuse étant fabriquée en un second matériau biocompatible ; et
dans lequel un médicament est transporté par la membrane poreuse à élution médicamenteuse.

10. Implant de système de conduction lymphatique selon la revendication 9, dans lequel le second matériau biocompatible de la membrane poreuse est un matériau résorbable qui inclut du PGA ou du PLGA.

11. Implant de système de conduction lymphatique selon l'une quelconque des revendications 9 à 10, dans lequel la membrane poreuse (14) est une maille de fibres électrofilées.

12. Implant de système de conduction lymphatique selon l'une quelconque des revendications 9 à 11, dans lequel le médicament est un médicament antibactérien, un médicament chimiothérapeutique et/ou un médicament antimétastasique.

13. Implant de système de conduction lymphatique selon l'une quelconque des revendications 9 à 12, dans lequel la paroi latérale tubulaire non poreuse (16) inclut de l'ePTFE.

14. Implant de système de conduction lymphatique selon l'une quelconque des revendications 9 à 13, dans lequel la paroi latérale tubulaire non poreuse (16) inclut un revêtement d'ePTFE.
